# EUROPÄISCHE PATENTANMELDUNG

(11) **EP 1 029 552 A1**
(43) Veröffentlichungstag der Anmeldung: **23.08.2000**
(21) Anmeldenummer: 00103554.2
(22) Anmeldetag: 18.02.2000
(51) Int. Cl.: A61L 2/18, A61L 2/22, A61L 9/14

(54) **Mittel, Verfahren, Vorrichtung sowie Verwendungen zum biologischen Entkeimen von Luft und Räumen mit Luft**

(30) Priorität: 18.02.1999 DE 19906843; 09.03.1999 DE 19910356
(71) Anmelder: Arconia GmbH, 7002 Chur (CH)
(72) Erfinder: Kern, Ralf M., 82057 Icking (DE)
(74) Vertreter: Kern, Ralf M., Dipl.-Ing.

(57) **Zusammenfassung**

Die Erfindung betrifft ein Mittel, ein Verfahren, eine Vorrichtung sowie Verwendungen zum biologischen Entkeimen von Luft und Räumen mit Luft, wobei die Raumluft entweder untoxisch und biologisch voll abbaubar entkeimt wird und auch laufend durch Zugabe bzw. Aufrechterhaltung der Wirkung eines biologisch völlig untoxischen Mittels in einem stark verkeimungsarmen Zustand gehalten werden kann.

Insbesondere kann das Verfahren dahingehend erweitert werden, daß der Luft eine geringe bzw.äußerst geringe Menge eines biologisch untoxischen Mittels zugegeben wird, daß die Luft selbst eine keimreduzierende Wirkung ausübt, also mindestens ein geringes eigenes Entkeimungspotential besitzt, wobei dieser keimreduzierende Anteil des Mittels in der Luft nicht nur völlig biologisch untoxisch, sondern auch geruchsmäßig nicht irgendwie spürbar ist.

## Beschreibung

Die Erfindung betrifft ein Mittel, ein Verfahren, eine Vorrichtung sowie Verwendungen zum biologischen Entkeimen von Luft und Räumen mit Luft.

Wenn man Luft und die Zuführung von Luft in Räume entkeimt, dann ist eine anhaltende keimreduzierte Konstitution dieser Luft allenfalls durch laufende Zuführung ausreichender Mengen von keimfreier bzw. keimreduzierter Luft begrenzt gewährleistet, weil innerhalb benutzter Räume entweder durch die Benutzer selber oder eine durch andere Einflüsse auftretende, rauminterne stetige Neuverkeimung eintritt, was insbesondere für eine intensive Raumbenutzung auch durch das Einschleppen von Keimen von außerhalb des Raumes gilt.

Man denke dabei nur an Krankenhäuser mit ihrer spezifischen Gefährdung durch pathogene Keime oder Versammlungs- oder Hotelräume, aber auch Lagerräume und Produktionsräume oder dergleichen.

Bei einer Entkeimung auf bekannte Weise ist es nicht möglich, die Luft biologisch weitgehend genug zu entkeimen, zumal diese von den sie wegen ihrer Zuführungen - z.B. Luftwäscher, Filter und Luftkanal-Zuführungen - die insbesondere bei warmer Luftatmosphäre stets erheblicher Neu-Verkeimung ausgesetzt ist, selbst wenn diese laufend keimfrei bzw. keimmindernd gereinigt werden.

Aber auch bei einer solchen intermittierenden Reinigung dürfen die Maßnahmen und die Reinigungsmittel nicht biologisch schädlich sein, weil Reste dieser Reinigung die neu durchgeleitete Luft nicht toxisch belasten dürfen.

Mit den bekannten Entkeimungsverfahren ist mithin ein erheblicher Energieverbrauch, sind laufender Reinigungsaufwand und Benutzungsausfälle verbunden und ist trotzdem eine laufend noch unerwünscht hohe Verkeimung als Kompromißlösung inkauf zu nehmen. Dieser Verkeimungsgrad kann bei besonders verkeimungsspezifischen Räumen, z.B. Krankenhäusern, trotz aller Reinigungsmaßnahmen hoch gesundheitsgefährlich sein.

Zur Lösung dieser Probleme bzw. Vermeidung dieser Nachteile liegt die Erfindung in einem Verfahren und einem Mittel und dessen Verwendung, bei dem die Raumluft laufend durch Zugabe bzw. Aufrechterhaltung der Wirkung eines biologisch völlig untoxischen Mittels in einem stark verkeimungsarmen Zustand gehalten wird.

Ein erheblich darüberhinausgehendes Verfahren liegt darin, daß der Luft eine geringe bzw. äußerst geringe Menge eines biologisch untoxischen Mittels zugegeben wird, daß die Luft selbst eine keimreduzierende Wirkung ausübt, also mindestens ein geringes eigenes Entkeimungspotential besitzt, wobei dieser keimreduzierende Anteil des Mittels in der Luft nicht nur völlig biologisch untoxisch, sondern auch geruchsmäßig nicht irgendwie spürbar ist.

Das Mittel kann durch übliche Luftbefeuchter mit und ohne Ventilator oder Luftreiniger, wie Luftwäscher oder andere Verdunster aus seiner in Wasser in geringer Konzentration gelösten Form überraschenderweise von der natürlichen Feuchtigkeit der Luft durch Aerosol-Versprühung, Verdunsten oder an mit dem Mittel bedeckten Oberflächen von der Luft bzw. deren relativer Luftfeuchtigkeit aufgenommen werden. Um die Luftbefeuchter oder die Eingangsfilter der Belüftungsanlagen selbst vor der Verkeimung zu bewahren und auch die Luft optimal von Verkeimung zu entlasten, ist es erfindungsgemäß vorteilhaft, die Luft bereits vor dem Eintritt in die Belüftungsanlagen gründlich mit dem Mittel keimreduzierend zu behandeln und gegebenenfalls nochmals vor dem Eintritt in den zu belüftenden Raum.

Dafür kann auch eine grundlegende Entkeimung von Klima-und Raumluftanlagen einfach durch Aufbringung des Mittels auf die Innenwände der Luftkanäle und/oder durch Verwendung von z.B. damit imprägnierten und nachimprägnierbaren Matten innerhalb der Kanäle erfolgen. Dies gilt auch für Krankenhaus- und Reinstlufträume.

Derartige Matten oder auch Tuche, Filze oder dergleichen vergrößern durch ihre Faser- und Kapillarwirkung die wirksame Verdunstungsoberfläche und verstärken den Verdunstungsübergang in die Luft insbesondere auch dann, wenn die Luft auf sie aufprallt und durch sie hindurch tritt. Etwaige Tröpfchen bei der Aerosolbefeuchtung werden dadurch kapillar verteilt und leichter verdunstet.

Bei der Verwendung auf verkeimten, insbesondere auch verpilzten Flächen, wie beispielsweise schimmelbefallenen Wänden, erfüllt das erfindungsgemäße Mittel auch eine Doppelfunktion, nämlich die dauerhafte Entkeimung der damit in Berührung kommenden Raumluft.

Das kann auch als Übertragung aus mit dem Mittel imprägnierten bzw. oberflächenmäßig versehenen Gegenständen (z.B. Gardinen, Teppichen oder Wänden oder dergleichen) geschehen.

Beispielsweise wird diese Wirkung auch als Beimischung zum Putz, in der Farbe oder in damit imprägnierten Wandbelägen erzielt.

Dabei ist es von zusätzlichem Vorteil, wenn die Luft in einem Zustand mit dem in wässrigem Medium gelösten Mittel in Kontakt gebracht wird, indem ihr Feuchtigkeitspartialdruck gering ist, die Luft also trockener als ihre Umgebung ist bzw. eine niedrige relative Luftfeuchtigkeit aufweist.

Das Mittel wird von der natürlichen Feuchtigkeit der Luft aber auch in angetrocknetem Zustand in feinster Konzentration, die noch keimreduzierend ist, aufgenommen.

In feuchtkalten Kühl- oder Kellerräumen erfolgt z.B. eine durchgreifende, grundlegende Pilz-Entkeimung auf und innerhalb der Wände, als auch eine Entkeimung des zugehörigen Raumes, wobei auch Tür- und Fensterdichtungen und versteckte Ecken erfasst werden.

Schließlich ist das Mittel aber auch zur entsprechenden Zugabe zu Abluft, insbesondere warmer und feuchter, dampfhaltiger Abluft einsetzbar, wobei es in niedriger Konzentration desodorierend und umweltschonend entkeimend wirkt und auch ein Zusetzen der Abluftanlagen mit Biofouling verhindert.

Dadurch, daß die keimreduzierende Wirkung der mittelbeaufschlagten Luft durch die kontinuierliche Luftzufuhr und -umwälzung erfolgt, wird die Verkeimung der Räume auf einem niedrigen Niveau gehalten, so daß auch der Aufwand für eine gegebenenfalls erwärmte Luftzufuhr und für die Heizung reduziert wird.

Die Erfindung liegt demgemäß auch in einem automatisch durchführbaren Verfahren zur Einstellung niedriger Verkeimung auch von laufend wiederverkeimten Räumen, wie z.B. Büro- und Hotelräumen, sowie starkem Publikumsverkehr ausgesetzten Räumen, indem im Bereich der Luftzufuhr zunächst gegebenenfalls unter Abkühlung und Taubildung und damit Verarmung der in der Luft enthaltenen Feuchtigkeit deren Luftfeuchtigkeit stark reduziert wird und danach mit dem entkeimenden Mittel wieder befeuchtet wird, so daß auf diese Weise ein hohes Entkeimungspotential aufgebaut wird.

In Lagerräumen läßt sich ein äußerst keimreduzierter Zustand der Luft durch Verwendung vergleichsweise trockener Luft kombiniert mit dem erfindungsgemäßen Mittel erreichen, und zwar bei nur niedriger, aber nicht unter 0°C liegender Lufttemperatur, wobei gegenüber Kühlhäusern eine erhebliche Energieeinsparung erfolgt.

Das für das erfindungsgemäße Verfahren mit dem genannten überraschenden Vorteilen - biologisch untoxisch (mit Trinkwasserklassifizierungs-Klasse 0) - und doch wirksam entkeimende Mittel macht grundsätzlich Gebrauch von der Anwendung einer synergistisch wirksamen Kombination eines oder mehrerer Lebensmittel-Konservierungsstoffe mit mindestens einer nicht-toxischen, insbesondere organischen Säure, wobei in vergleichsweise geringen Konzentrationen in Wasser eine überraschend starke entkeimende bzw. keimreduzierende Wirkung eintritt.

An Einzelkomponenten dieses Mittels kommen die in Anspruch 2 genannten Verbindungen infrage, die mit einem Oxidationsmittel nach Anspruch 3 ergänzt sein können.

Vorzugsweise haben sich Wirkstoff-Kombinationen nach einem der Ansprüche 4 und 5 und in einer wässrigen Lösung in einer Verdünnung nach Anspruch 6 in der Konzentrationszusammenstellung nach den Ansprüchen 7 und 8 mit einem PH-Wert zwischen 2 und 7, also von leicht sauer (<7,2) bis sauer bewährt.

Zur Senkung der Oberflächenspannung der wässrigen Lösung des Mittels kann die Zugabe von 0,1 bis 5 % eines Tensids nach Anspruch 10 vorgesehen sein und zum Schutz der betroffenen Anlagen ein Korrosionsschutzmittel oder eine Oberflächenschutzkomponente nach Anspruch 12 oder 13.

Mit dem erfindungsgemäßen Verfahren nach den Ansprüchen 14 bis 18 wird die erfindungsgemäße, unerwartet wirksame Lösung der vorgenannten neuen Aufgabe mit den eingangs genannten Vorteilen bzw. Wirkungen erreicht.

Das Mittel wirkt gegen Keime wie Bakterien (bakterizid), Pilze wie Schimmelpilze (fungizid), Pilzsporen und Blütenstaub, Hefen, Protozoen, Eukaryoten, Eiweißmoleküle, Enzyme, Makrophagen und auch molekularbiologische Schädlinge oder dergleichen, niedrige Pflanzen wie Algen sowie Protozoen (Einzeller), aber auch gegen Kleinlebewesen, Insekten, Milben, Parasiten durch Zerstörung deren Nachwuchs und Eier, sowie Biofouling und Epelithionbildung.

Konkret ist das erfindungsgemäße Mittel auch gegen sämtliche Arten von Aspergillus, Legionellen und Salmonellen, sowie
Escherichia coli,
Klebsielle pneumoniae,
Staphylococcus aureus,
Pseudomonas aeruginosa,
Streptococcus faecalis,
Proteus mirabilis,
Mycobacterium tuberculosis,
Clostridium sporogenes,
Candida albicans
mit völliger Entkeimung erprobt.

Das Mittel ist nicht nur völlig untoxisch, sondern auch 100 % biologisch abbaubar und praktisch gegen jede Art von Geruchsbildung wirksam.

Mit der Erfindung ist auch keinerlei Resistenzerscheinung zu befürchten

Eine spezifische Vorrichtung kann außer in jeder Art von Luftbefeuchtern und Aerosoleinrichtungen auch in einem Wandanstrich bestehen, dessen Farbe das Mittel in gegebenenfalls stärkerer Konzentration in langanhaltender Wirkung beigesetzt ist, an der einerseits die Raumluft keimreduziert wird und andererseits die Wände prophylaktisch gegen Schimmelpilzbefall geschützt sind.

Auch können geeignete Gegenstände in den Räumen mit dem Mittel versehen sein, da es die Oberflächen bzw. das Material der Gegenstände nicht angreift.

Das erfindungsgemäße Mittel kann außer als anhaltend und gründlich wirksames Reinigungsmittel für alle die Luft führenden Konstruktionselemente, auch für spezifisch geruchsbelastete Räume wie beispielsweise Leichenaufbewahrungs-Räume verwendet werden.

Die Erfindung ist nicht nur für die Lagerung von Trockenobst und Gewürzen, sondern auch für den Trocknungsvorgang selbst mit großen Vorteilen anwendbar, indem dieser mit keimfreier bzw. keimreduzierter Luft erfolgt.

Mit der Erfindung ist es zudem möglich, in Reinsträumen für Chip-Herstellung die bisher nicht zu bewältigende Restverkeimung von 100 und 10 Keimen je Bezugseinheit noch weiter zu reduzieren.

Letztendlich wird mit der Erfindung auch eine laufende Keimreduzierung auf der z.B. menschlichen Haut einschließlich der Schleimhäute und Lungen erreicht, womit auch eine Entlastung des Immunsystems erfolgt, so daß es vor Überbeanspruchung bewahrt wird.

## Patentansprüche

1. Mittel zum biologischen Entkeimen von Luft und Räumen mit Luft mit einem oder mehreren Lebensmittel-Konservierungsstoffen sowie mindestens einer nichttoxischen, insbesondere organischen Säure.

2. Mittel nach Anspruch 1, welches mindestens eine Kombination einer oder mehrerer Verbindungen aus der Gruppe der Verbindungen Citronensäure, Sorbinsäure, L-Ascorbinsäure und/oder Acetylsalicylsäure oder deren Derivate aufweist
mit mindestens einem Lebensmittelkonservierungsstoff wie beispielsweise Benzoesäure und deren Derivate wie
Sorbinsäure, Citronensäure, Weinsäure, Milchsäure, Apfelsäure, Essigsäure, Bernsteinsäure, Acetysalicyl-und L-Ascorbinsäure sowie
Natriumbenzoat, Hydroxybenzoesäure, Ameisensäure, Propionsäure und Abkömmlinge sowie Salze, Ester und/oder Mischungen derselben.

3. Mittel nach Anspruch 1, dadurch gekennzeichnet, daß es einen Anteil eines Oxidationsmittels wie eine Perverbindung aus z.B. einem Peroxid, wie Natrium-, Kalium-, Calcium- oder Wasserstoffperoxid oder einen Abkömmling einer Persäure wie Perborat, Perphosphat, Percarbonat oder eine stark oxidierende Verbindung wie ein Permanganat aufweist.

4. Mittel nach einem der Ansprüche 1 bis 3, dadurch gekennzeichnet, daß die Kombination von 50 bis 80 Gew.%, bevorzugt 55 bis 70 Gew.%, insbesondere 60 bis 65 Gew.% Citronensäure, 20 bis 50 Gew.%, bevorzugt 30 bis 45 Gew.%, insbesondere 35 bis 40 Gew.% einer festen Peroxoverbindung, sowie 0,3 bis 1 Gew.% eines Lebensmittelkonservierungsstoffes aufweist, wobei die Summe aller Prozentsätze jeweils 100 ist.

5. Mittel nach Anspruch 4, dadurch gekennzeichnet, daß die Citronensäure bis zu 1 bis 25 Gew.% der Gesamtmenge durch L-Ascorbinsäure und/oder Acetylsalicylsäure ersetzt ist.

6. Mittel nach einem der Ansprüche 1 bis 5 in wässriger Lösung von 15 - 0,03 g Mittel, bevorzugt 10 - 0,1 g, insbesondere 8 - 0,1 g Mittel pro Liter Wasser.

7. Mittel nach einem der Ansprüche 1 bis 5, gekennzeichnet durch 0,05 - 10 g, bevorzugt 0,2 - 6 g und besonders bevorzugt 0,3 - 4 g Natriumbenzoat und 0,1 - 10 g, bevorzugt 0,2 - 5 g und besonders bevorzugt 0,3 - 3 g Sorbinsäure.

8. Mittel nach einem der Ansprüche 1 bis 6 mit zusätzlich 0,001 - 16, vorzugsweise 12, insbesondere 10 Vol.-% H₂O₂ pro Liter Wasser sowie gegebenenfalls Stabilisatoren für die Peroxidverbindung.

9. Mittel nach einem der vorhergehenden Ansprüche 1 bis 6, dadurch gekennzeichnet, daß die Lösung einen sauren PH-Wert zwischen etwa 2 und 7 aufweist.

10. Mittel, insbesondere nach einem der vorhergehenden Ansprüche, gekennzeichnet durch 0,1 bis 5 % eines oder mehrerer Tenside ausgewählt aus der Gruppe bestehend aus in der verwendeten Menge untoxischen anionischen und nichtionischen Tensiden, Seifen, Fettsäure-Aminocarbonsäurekondensate, Alkylbenzolsulfonate, Ligninsulfonate, Alkylsulfonate, Fettsäureestern, sulfatierten Fettsäureglyceriden, Betaine, Sulfobetaine, Saponine, Fettsäureester von Polyalkoholen, Oxyäthylierte Fettsäuren, etc., und insbesondere Natriumdodecylsulfonat und Natriumlaurylsulfat.

11. Mittel nach einem der vorhergehenden Ansprüche, dadurch gekennzeichnet, daß die organischen Säuren gesättigte oder ungesättigte Carbonsäuren mit 1-14 C-Atomen, bevorzugt 1-12 C-Atomen sind.

12. Mittel nach einem der vorhergehenden Ansprüche, dadurch gekennzeichnet, daß ihm ein Korrosionsschutzmittel vorzugsweise in 0,1 bis 1 Gew.% in der bei der Behandlung verwendeten Konzentration der Lösung zugesetzt ist.

13. Mittel nach einem der vorhergehenden Ansprüche, dadurch gekennzeichnet, daß ihm eine Oberflächenschutzkomponente, z.B. Wachs beigefügt ist.

14. Wandputz oder Anstrichmittel, dadurch gekennzeichnet, daß es mit dem Mittel nach den Ansprüchen 1 bis 13 versetzt ist.

15. Verfahren zum Entkeimen von Raum- und Abluft unter Verwendung insbesondere des Mittels nach den Ansprüchen 1 bis 13, dadurch gekennzeichnet, daß die Raumluft dem Mittel in geringer Konzentration zur Aufnahme des Mittels in der Luft ausgesetzt wird.

16. Verfahren nach Anspruch 15, dadurch gekennzeichnet, daß die Raumluft in einem Maße mit dem Mittel nach den Ansprüchen 1 bis 13 angereichert wird, bei dem die Raumluft laufend durch Zugabe bzw. Aufrechterhaltung der Wirkung eines biologisch völlig untoxischen Mittels in einem stark verkeimungsarmen Zustand gehalten wird, so daß die Raumluft selbst als keimreduzierendes Mittel wirkt.

17. Verfahren nach Anspruch 15, dadurch gekennzeichnet, daß die Raumluft vor der Behandlung mit dem Mittel getrocknet wird.

18. Verfahren nach Anspruch 15, dadurch gekennzeichnet, daß die Behandlung mit dem Mittel bei erhöhter Temperatur erfolgt.

19. Verwendung des Mittels insbesondere nach den Ansprüchen 1 bis 13 und dem Verfahren nach den Ansprüchen 15 bis 18 für Lagerräume.

20. Verwendung des Mittels insbesondere nach den Ansprüchen 1 bis 13 und dem Verfahren nach den Ansprüchen 15 bis 18 für Reinstlufträume.

21. Verwendung des Mittels insbesondere nach den Ansprüchen 1 bis 13 für die Herstellung von Trockenprodukten wie Obst und Gemüse.

22. Verwendung des Mittels insbesondere nach den Ansprüchen 1 bis 13 für die Oberflächenentkeimung.

23. Verwendung des Mittels insbesondere nach den Ansprüchen 1 bis 13 zur Beimischung zu Oberflächenbedeckungen wie z.B. Farben, Putz und Estrich.
